# EUROPÄISCHE PATENTANMELDUNG

(11) **EP 2 075 024 A1**
(43) Veröffentlichungstag der Anmeldung: **01.07.2009**
(21) Anmeldenummer: 08022115.3
(22) Anmeldetag: 19.12.2008
(51) Int. Cl.: A61M 16/04, A61M 25/10

(54) **Befülladapter zum Befüllen eines Cuffs**

(30) Priorität: 21.12.2007 DE 102007062861
(71) Anmelder: Frick, Ulrich, 71384 Weinstadt (DE)
(72) Erfinder: Frick, Ulrich, 71384 Weinstadt (DE)
(74) Vertreter: Kesselhut, Wolf

(57) **Zusammenfassung**

Die Erfindung betrifft einen Befülladapter (1) zum Befüllen eines Cuffs, der an einem in die menschliche Trachea einsetzbaren Tubus zur Abdichtung desselben angeordnet und über einen Zuleitungsschlauch (2) mit Luft beaufschlagbar ist. Der Adapter zeichnet sich durch ein nach außen hin geschlossenes Gehäuse (4) mit einem darin definierten Überdruckbereich (6), erste mit dem Überdruckbereich (6) in Strömungsverbindung stehende und mit dem Zuleitungsschlauch (2) druckdicht verbindbare auslassseitige Anschlussmittel (8a, 8b), zweite einlassseitige Anschlussmittel (12a, 12b), die über eine Spritze (14) mit Überdruck beaufschlagbar sind und die mit dem Überdruckbereich (6) über ein erstes Ventil (10) strömungmäßig verbindbar sind, sowie ein mit dem Überdruckbereich (6) strömungsmäßig verbundenes Überdruckventil (30), welches den Überdruckbereich (6) beim Überschreiten eines vorgegebenen Drucks entlüftet, aus.

## Beschreibung

Die Erfindung betrifft einen Befülladapter zum Befüllen eines Cuffs, der insbesondere an einem in die menschliche Trachea einsetzbaren Tubus zur Abdichtung derselben angeordnet ist, gemäß dem Oberbegriff von Anspruch 1.

Bei den heutzutage in der Medizin eingesetzten Tracheostomiekanülen und Endotrachealtuben, die beispielsweise zur künstlichen Beatmung von Patienten in deren Trachea eingesetzt werden, ist an deren Spitze ein blasen- oder ballonförmiger Abschnitt angebracht, der beim Einführen in die Trachea flach an der Tubuswand anliegt und nach dem Einführen mit Luft beaufschlagt werden kann, um beispielsweise im Falle einer künstlichen Beatmung sicherzustellen, dass der unterhalb des Cuffs liegende Lungenabschnitt abgedichtet ist. Hierdurch wird sichergestellt, dass kein Sekret, Blut oder Erbrochenes etc. in die Lunge aspiriert werden kann, wodurch die Gefahr eines Hustenreizes oder gar einer Lungenentzündung deutlich reduziert wird. Zudem wird durch den expandierten ballonartigen Abschnitt sichergestellt, dass im Falle einer künstlichen Beatmung der zugeführte Sauerstoff nicht teilweise am Tubus vorbei nach außen entweichen kann. Aus Gründen der sprachlichen Vereinfachung werden die Tracheostomiekanülen und Endotrachealtuben nachfolgend als Tubus, und der daran angeordnete blasenförmige expanierbare Abschnitt gemäß dem üblichen Fachterminus als Cuff bezeichnet.

Darüber hinaus werden Cuffs ebenfalls im Bereich der Gefäßmedizin, z.B. zur Stillung von Blutungen nach Operationen, eingesetzt.

Um den die Außenwand des Tubus ringförmig umgebenden Cuff nach dem Einsetzen mit Luft zu beaufschlagen, ist im Inneren des Tubus ein Zuleitungsschlauch entlang geführt, der mit seinem einen Ende mit dem Cuff verbunden ist, und dessen anderes Ende mit einem Sperrventil, beispielsweise einem federbelasteten Einwegventil sowie einem entsprechenden Anschlussadapter oder Anschlusskonnektor zur Aufnahme des konischen männlichen Anschlusskonnektors einer bekannten Einwegspritze verbunden ist.

Um den Cuff nach dem Einführen des Tubus in die Trachea des Patienten zu expandieren, wird eine zuvor erwähnte Einwegspritze auf den Adapter aufgesteckt und durch Verschieben des Spritzenkolbens so lange Luft durch das Einwegventil hindurch in den Cuff eingebracht, bis dieser mehr oder minder stark befüllt ist und an der Innenwand der Trachea anliegt. Anschließend wird die Spritze abgezogen und die Zuleitung durch das Einwegventil geblockt, so dass der Druck innerhalb des Cuffs aufrecht erhalten bleibt

Da die Geometrie und der Innendurchmesser der Trachea eines Patienten je nach Alter, Geschlecht, Größe des Patienten etc. individuell verschieden sind, und sich die von den unterschiedlichen Herstellern der Cuffs eingesetzten ballonartigen Blasen je nach Kanülen- und Tubusgröße sowie Tubusform in ihrer Größe und Form unterscheiden, ergibt sich häufig das Problem, dass die Cuffs mit einem zu hohen oder einem zu niedrigen Druck befüllt werden.

Das zuvor geschilderte Problem wird dadurch noch verstärkt, dass die üblichen Einwegspritzen häufig sehr unterschiedliche Betätigungskräfte erfordern, da die Passung zwischen dem Spritzenkolben und der Innenwand des zylindrischen Spritzenkörpers sowie auch das Gleitverhalten sehr unterschiedlich sein können und somit selbst bei geübten Benutzern das Beaufschlagen der Cuffs mit einem ordnungsgemäßen Überdruck ohne zusätzliche geeignete Messinstrumente in der Regel nicht möglich ist.

Da es bei einem zu hohen Druck innerhalb des Cuffs zu irreparablen Gewebeschädigungen kommen kann; und bei einem zu niedrigen Druck eine zuverlässige Abdichtung des Tubus innerhalb der Trachea nicht mehr gewährleistet ist - wodurch es zu der zuvor erwähnten Aspiration von Sekret und Erbrochenem kommen kann - werden heutzutage oftmals aufwendige Handmessgeräte verwendet, mit denen der Luftdruck ähnlich einem manuellen Blutdruckmessgerät durch Öffnen und Schließen eines entsprechenden Ventils und Überprüfen mittels eines Manometers auf einen gewünschten Wert abgelassen oder auch erhöht werden kann.

Abgesehen davon, dass die hierfür erforderlichen Geräte vergleichsweise aufwändig und teuer sind, besteht zudem das Problem, dass die Handhabung derselben besonders geschultes Personal voraussetzt, um Bedienungsfehler auszuschließen.

Weiterhin sind elektronisch gesteuerte Cuff-Druckmessgeräte bekannt, die im Wesentlichen ebenfalls die Funktion eines manuell betätigten Handmessgerätes übernehmen und den Überdruck innerhalb des Cuffs zusätzlich permanent überwachen und steuern. Auch diese Geräte sind vergleichsweise aufwändig, noch teuerer als die zuvor genannten Geräte und bedürfen zudem einer besonderen Schulung des Personals, um zu verhindern, dass es im Falle einer Fehlbedienung zu gravierenden gesundheitlichen Schädigungen des Patienten kommt.

Demgemäß ist es eine Aufgabe der vorliegenden Erfindung, einen Befülladapter zum Befüllen eines Cuffs zu schaffen, der vergleichsweise einfach aufgebaut ist und auch von ungeschultem Personal in der bisher bekannten Weise mittels einer manuell betätigten Luftfördereinrichtung, insbesondere einer Einwegspritze oder einem Gummibalg ohne weitere zusätzliche Hilfseinrichtungen und Messgeräte mit hoher Präzision mit einem vorgegebenen Druck befüllt werden kann.

Diese Aufgabe wird erfindungsgemäß durch einen Befülladapter mit den Merkmalen von Anspruch 1 gelöst.

Der erfindungsgemäße Befülladapter besitzt den Vorteil, dass dieser eine geringe Baugröße aufweist, mechanisch robust ist und in sehr kostengünstiger Weise mit einfachsten Mittel gefertigt werden kann. Zudem ist es ein weiterer Vorteil, dass für den Betrieb des Befülladapters keine elektrische Energie erforderlich ist, wodurch sich dieser beispielsweise im Falle von Katastrophen wie Erdbeben oder dergleichen auch außerhalb von Krankenhäusern oder auch in der Notfallmedizin von nicht speziell geschultem Personal einsetzen lässt.

Die Erfindung wird nachfolgend mit Bezug auf die Zeichnungen anhand einer bevorzugten Ausführungsform beschrieben.

In den Zeichnungen zeigen:
- Fig. 1: eine schematische Querschnittsansicht der bevorzugten Ausführungsform des erfindungsgemäßen Befülladapters mit einer Teildarstellung des Zuleitungsschlauchs zum Befüllen eines Cuffs zu Beginn des Befüllvorgangs,
- Fig. 2: den Befülladapter von Fig. 1 nach dem Aufstecken auf den konischen weiblichen Anschlusskonnektor des Zuleitungsschlauchs und nach dem Aufstecken einer teilweise dargestellten Einwegspritze in die zweiten einlassseitigen Anschlussmittel des Adapters,
- Fig. 3: den erfindungsgemäßen Befülladapter von Fig. 2 nach dem Verschieben der Spritze zur Aktivierung des den einlassseitigen Anschlussmitteln zugeordneten Ventils und Verschieben des Kolbens der Einwegspritze während des Befüllens des Cuffs mit Luft aus der Einwegspritze,
- Fig. 4: den Befülladapter von Fig. 3 nach dem Erreichen des vorgegebenen Überdruck-Sollwertes im Überdruckbereich des Gehäuses mit schematisch angedeutetem geöffnetem Überdruckventil,
- Fig. 5: den auf den weiblichen Anschlusskonnektor des Zuleitungsschlauchs aufgesteckten Befülladapter nach Abschluss des Befüllvorgangs und verschließen des Auf-/Zuventils, und
- Fig. 6: den erfindungsgemäßen Befülladapter von Fig. 5 während der Expansion des luftballonartigen Expansionsbehälters zum Abbau eines kurzzeitigen Überdruckstoßes im Überdruckbereich, der durch einen Hustenreiz hervorgerufen ist.

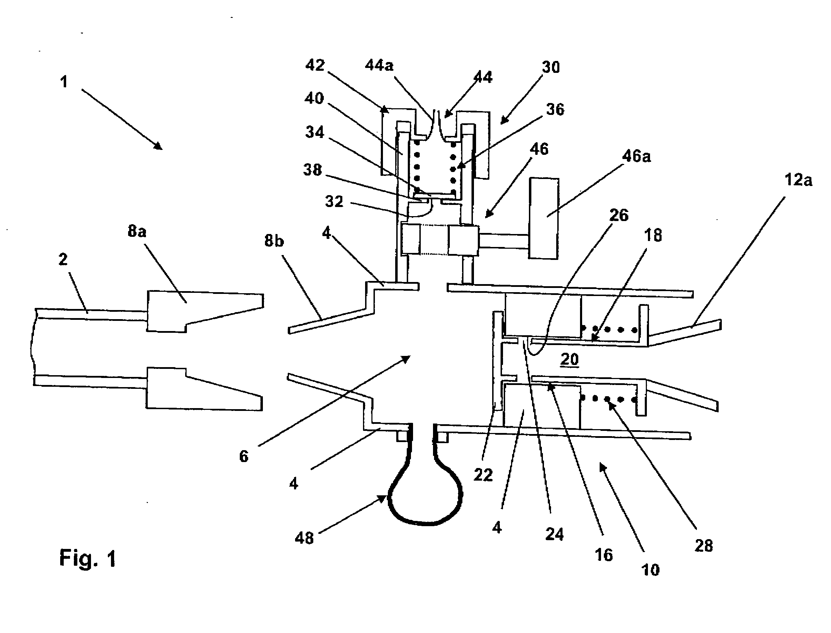

Wie in den Figuren 1 bis 6 gezeigt ist, umfasst ein erfindungsgemäßer Befülladapter 1 zum Befüllen eines in den Zeichnungen aus darstellungstechnischen Gründen nicht näher gezeigten Cuffs, der an einem in die menschliche Trachea einsetzbaren, ebenfalls nicht gezeigten Tubus zur Abdichtung desselben angeordnet und über einen abschnittsweise dargestellten Zuleitungsschlauch 2 mit Druckluft beaufschlagbar ist, ein nach außen hin im Wesentlichen geschlossenes Gehäuse 4, welches beispielsweise aus einem in der Medizin verwendeten Kunststoffmaterial, beispielsweise dem Kunststoffmaterial aus welchem Einwegspritzen gefertigt sind, besteht.

Wie in den Figuren weiterhin gezeigt ist, ist in dem Gehäuse 4 ein Überdruckbereich 6 definiert, der nach dem Aufstecken des erfindungsgemäßen Befülladapters 1 auf einen insbesondere weiblichen Anschlusskonnektor 8a am Zuleitungsschlauch 2 mit dem Cuff in Strömungsverbindung steht. Hierzu sind am Gehäuse 4 erste auslassseitige Anschlussmittel, beispielsweise in Form des gezeigten männlichen konischen Anschlusskonnektors 8b, geformt, welche zur Erzeugung einer druckdichten Verbindung in bekannter Weise in den zugehörigen konischen weiblichen Anschlusskonnektor 8a eingesteckt und in diesem aufgrund der Konizität der Anschlusskonnektoren 8a, 8b reibschlüssig fixiert werden, um gewünschtenfalls eine dauerhafte druckdichte Verbindung zwischen dem Zuleitungsschlauch und dem Befülladapter 1 zu erzeugen.

In Gleicher Weise ist es jedoch gemäß einer in den Figuren nicht gezeigten Ausführungsform möglich, dass der Zuleitungsschlauch 2 direkt auf einen männlichen Anschlusskonnektor 8b des Gehäuses 4 aufgesteckt, und z.B. durch Verkleben oder durch Verklemmen dauerhaft an diesem fixiert wird. Hierbei kann der männliche Anschlusskonnektor 8b z.B. auch die Form eines zylindrischen Röhrchens aufweisen, wodurch sich die Fertigung gegenüber der in den Figuren gezeigten konischen Ausführungsform zusätzlich vereinfachen lässt.

Zudem kann es vorgesehen sein, dass im weiblichen Anschlusskonnektor 8a oder im Zuleitungsschlauch 2 ein bekanntes und in den Figuren nicht gezeigtes Einwegventil aufgenommen ist, welches durch Einstecken des männlichen auslasseitigen Anschlusskonnektors 8b mechanisch geöffnet und anschließend durch den eingesteckten männlichen Anschlusskonnektor 8b in der geöffneten Stellung gehalten wird.

Gemäß der Darstellung von Fig. 1 ist auf der den auslassseitigen Anschlussmitteln 8b gegenüberliegenden Seite des Überdruckbereichs 6 ein erstes Ventil 10 angeordnet, das mit zweiten einlasseitigen Anschlussmitteln versehen ist, welche bevorzugt die Form eines konischen weiblichen Anschlusskonnektors 12a besitzen, in welchen ein entsprechend ausgebildeter konischer männlicher Anschlusskonnektor 12b einer bekannten manuell betätigten Luftfördereinrichtung, insbesondere einer Einwegspritze 14, wie in den Figuren 1 bis 4 gezeigt, oder auch eines Gummibalgs, druckdicht einsteckbar ist.

Obgleich als Luftfördereinrichtung jedwede bekannte, von Hand betätigte Pumpe zum Einsatz gelangen kann, wird die Erfindung nachfolgend der Einfachheit halber am Beispiel einer bekannten Einwegspritze 14 beschrieben.

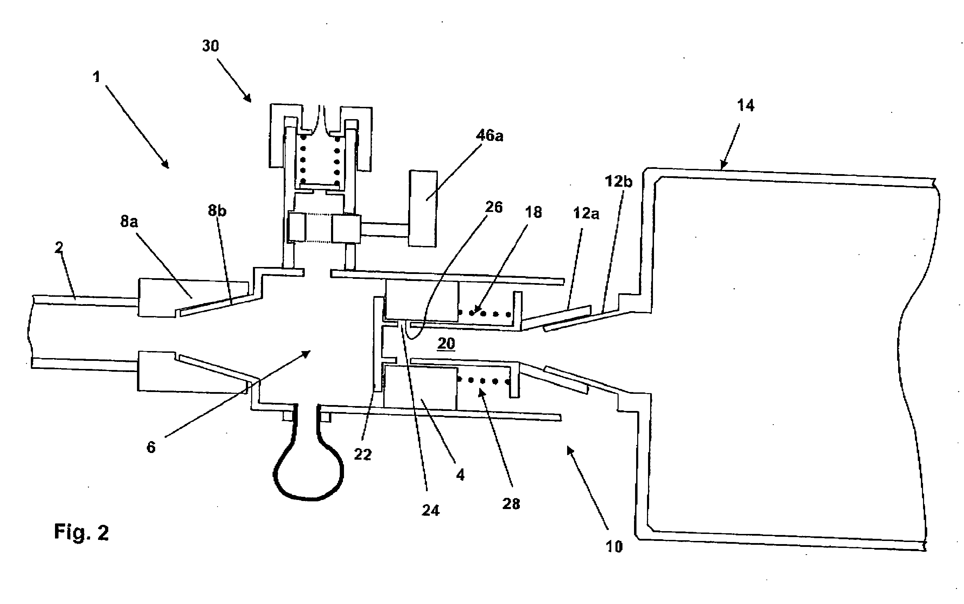

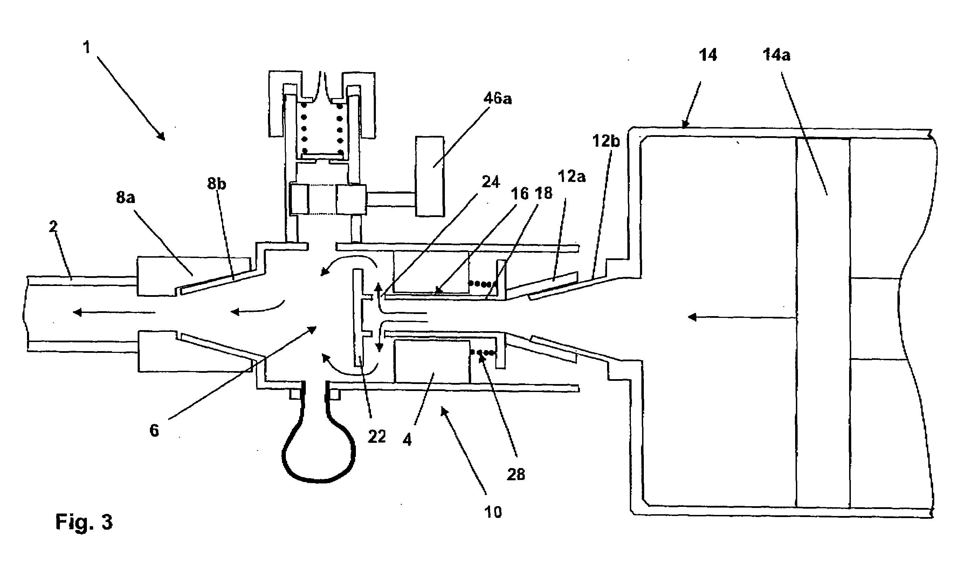

Wie hierbei durch die Unterschiede in den Darstellungen der Figuren 2 und 3 angedeutet ist, besitzt das erste Ventil 10 einen in einer Bohrung 16 des Gehäuses 4 verschiebbar aufgenommenen zylindrischen Hohlkörper 18, dessen Innenraum 20 an seinem dem Überdruckbereich 6 zugewandten Ende stirnseitig durch eine Platte oder einen Flansch 22 verschlossen ist. Demgegenüber ist der zylindrische Hohlkörper 18, der bevorzugt ebenfalls aus Kunststoff gefertigt ist, an seinem anderen Ende mit den zweiten einlassseitigen Anschlussmitteln 12a verbunden. Letztere sind vorzugsweise integral mit dem Hohlkörper 18 geformt.

In der zylindrischen Außenwand des Hohlkörpers 18 ist in einem Abstand von z. B. 1 bis 2 mm von der Begrenzungswand des Überdruckbereichs 6 entfernt eine radiale Durchgangsbohrung 24 gebildet, welche in der beispielsweise in Fig. 1 und 2 gezeigten Position durch die entsprechende innere Wandfläche 26 der Bohrung 16 verschlossen wird, wenn sich der zylindrische Hohlkörper 18 in der in Fig. 1 und 2 gezeigten Schließstellung befindet.

Wie den Zeichnungen hierbei weiterhin entnommen werden kann, stützt sich der Hohlkörper 18 bevorzugt über federelastische Mittel in Form einer Spiraldruckfeder 28 am inneren Teil des Gehäuses 4 ab, welche den Hohlkörper 18 in die in den Figuren 1, 2 und 6 gezeigte Schließstellung drängen.

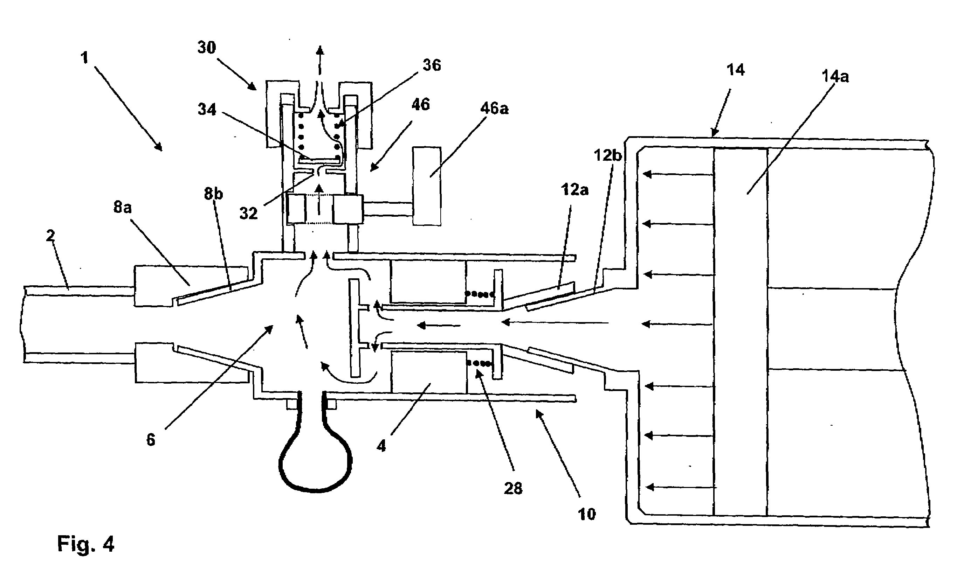

Gemäß der Darstellung von Fig. 3 und 4 lässt sich der Hohlkörper 18 nach dem Einführen des männlichen Anschlusskonnektors 12b einer Einwegspritze 14 durch Bewegen der Spritze 14 relativ zum Gehäuse 4 entgegen der Kraft der federelastischen Mittel 28 innerhalb der Bohrung 16 verschieben, wodurch die radiale Bohrung 24 aus dem dichtenden Innenwandbereich der Bohrung 16 des Gehäuses 4 herausbewegt wird. Dies wiederum führt dazu, dass für die aus der Einwegspritze 14 zugeführte Luft ein Durchtritt geschaffen wird, über welchen die in den Zeichnungen durch Pfeile angedeutete Luft in den Überdruckbereich 6 und von dort aus über die Anschlusskonnektoren 8a und 8b sowie den Zuleitungsschlauch 2 in den Cuff eingebracht werden kann.

Nachdem der Cuff mit einer vorgegebenen Luftmenge befüllt ist und der Druck der Luft im Cuff bevorzugt im Bereich des Gegendrucks der Blutgefäße in der Trachea liegt, steigt der Druck im Inneren des Überdruckbereichs 6 mit fortschreitender Bewegung des Kolbens 14a der Spritze 14 weiter an.

Um in erfindungsgemäßer Weise sicherzustellen, dass der gewünschte Drucksollwert innerhalb des Überdruckbereichs, und damit der Druck innerhalb des Cuffs nicht überschritten wird, ist am Gehäuse 4 in erfindungsgemäßer Weise weiterhin ein Überdruckventil 30 vorgesehen, dass in Strömungsverbindung mit dem Überdruckbereich 6 steht.

Das Überdruckventil 30 ist vorzugsweise ein federbelastetes Einwegventil oder federbelastetes Rückschlagventil, welches eine Öffnung 32 aufweisen kann, die durch ein Plättchen oder eine Membran 34 verschlossen wird. Letztere wird hierbei gemäß der Darstellung in den Zeichnungen durch federelastische Mittel, z. B. in Form einer Spiraldruckfeder 36, dichtend gegen die Wandung 38 des Gehäuses 40 des Ventils 30 gedrängt, solange der Druck innerhalb des Überdruckbereichs 6 unterhalb des Drucksollwertes liegt. Wenn der Druck innerhalb des Überdruckbereichs 6 den Drucksollwert überschreitet, wird das Plättchen 34, wie in Fig. 4 gezeigt, entgegen der Kraft der Spiralfeder 36 von der Öffnung 32 abgehoben und der Überdruckbereich entlüftet.

Wie der Darstellung der Figuren 1 bis 6 in diesem Zusammenhang im Detail entnommen werden kann, stützt sich die Spiraldruckfeder 36 bevorzugt an einer das Gehäuse 40 übergreifenden Überwurfmutter 42 ab, welche über im Detail nicht näher gezeigte Gewindegänge verdrehbar am Gehäuse 40 des Ventils aufgenommen ist Die Überwurfmutter 42 übergreift dabei mit einem Teilabschnitt den inneren Bereich des Gehäuses 40 im Bereich der Spiraldruckfeder 36, so dass durch ein Verdrehen der Überwurfmutter 42 die Vorspannung der Spiraldruckfeder und hierdurch der Wert des Drucksollwertes, bei welchem das Überdruckventil 30 öffnet, verändert werden kann, um diesen beispielsweise um einen gewünschten Betrag zu erhöhen oder zu erniedrigen.

Demgemäß bilden die Spiraldruckfeder 36 und die über ein Gewinde verdrehbare Überwurfmutter 42, die bevorzugt mit einer nicht näher gezeigten Skala versehen ist, optionale Druck-Verstellmittel, mit denen sich die Größe des Drucksollwertes oder Öffnungsdrucks, bei welchem das Ventil 30 öffnet, verändern, bzw. das Ventil 30 kalibrieren lässt.

Nach einem weiteren der Erfindung zu Grunde liegenden Gedanken kann es vorgesehen sein, innerhalb des Gehäuses 40 des Überdruckventils 30 eine Wareneinrichtung 44 vorzusehen, die beim Öffnen des Überdruckventils 30 betätigt wird, um dem Bediener des erfindungsgemäßen Befülladapters 1 zu signalisieren, dass der gewünschte Überdruck im Cuff, erreicht ist. Obgleich die Warneinrichtung 44 bevorzugt eine mit dem Überdruckventil 30 in Strömungsverbindung stehende akustische Warneinrichtung ist, welche beispielsweise zwei in den Figuren schematisch angedeutete Plättchen oder Membranzungen 44a umfassen kann, die beim Durchströmen von Luft aus dem Überdruckventil 30 einen Pfeifton erzeugen, ist es ebenfalls denkbar, dass die Warneinrichtung zusätzlich oder optional eine in den Zeichnungen nicht näher gezeigte optische Anzeige umfasst, anhand von welcher der Bediener ablesen kann, ob der Druck innerhalb des Überdruckbereichs 6 den gewünschten Drucksollwert beim Befüllvorgang erreicht hat, bzw. anhand von welcher z.B. das Pflegepersonal erkennen kann, ob der Druck im Überdruckbereich 6 durch Undichtigkeiten z.B. über einen längeren Zeitraum hinweg unter den Drucksollwert abgefallen ist.

Denkbar ist in diesem Zusammenhang ein in den Zeichnungen nicht gezeigtes, mit dem Überdruckbereich 6 unmittelbar verbundenes durchsichtiges Röhrchen, in welchem ein Kolben mit einer abschnittesweisen farbigen Markierung, beispielsweise rot und grün, entgegen der Kraft einer Feder verschiebbar ist, derart, dass beim Unterschreiten oder auch Überschreiten des zulässigen Druckwertes im Überdruckbereich 30 der rote Abschnitt der Markierung sichtbar wird, wohingegen beim Erreichen des gewünschten Drucksollwerts der grüne Abschnitt sichtbar ist.

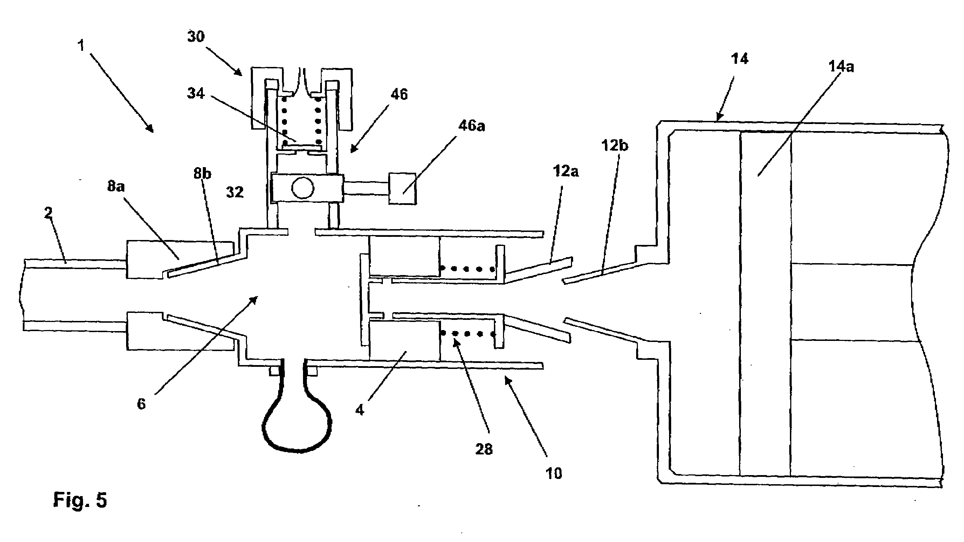

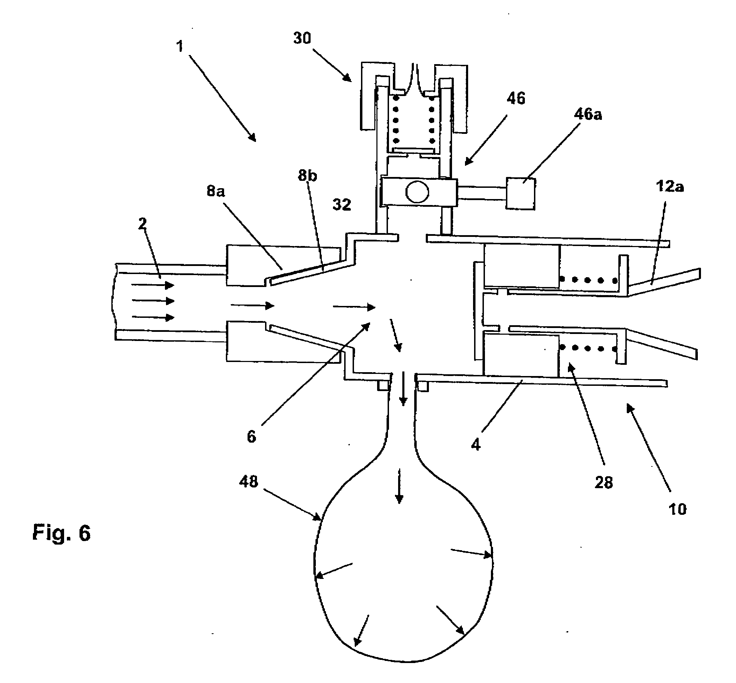

Wie den Darstellungen der Figuren 1 bis 6 weiterhin entnommen werden kann, ist bei der bevorzugten Ausführungsform der Erfindung dem Überdruckventil 30 in vorteilhafter Weise ein Absperrelement zugeordnet, welches bevorzugt als ein gezeigtes Auf-Zu-Ventil 46 ausgestaltet ist, das beispielsweise über den lediglich schematisch angedeuteten Hebel 46a betätigt wird und dem Überdruckventil 30 strömungmäßig bevorzugt vorgeordnet ist. Das Auf-Zu-Ventil 46 ist in den Figuren lediglich beispielhaft gezeigt und nicht auf die dort gezeigte Ausführungsform beschränkt. Das Absperrelement 46 dient dazu, nach dem Befüllen des Cuffs mit dem gewünschten Druck die Möglichkeit zu eröffnen, ein Entweichen von Luft aus dem Überdruckbereich 30 auch über einen längeren Zeitraum hinweg zuverlässig zu verhindern, indem der Hebel 46a aus der in den Figuren 1 bis 4 gezeigten Durchgangsstellung für das Ventil 46 in die in Fig. 5 und 6 gezeigte SperrStellung verdreht wird.

Durch den Einsatz des zuvor beschriebenen Absperrelements 46 wird zudem in vorteilhafter Weise gewährleistet, dass bei einem Hustenreiz des Patienten das Überdruckventil 30 durch den kurzzeitigen sehr starken Druckanstieg im Cuff nicht betätigt wird und der Druck im Überdruckbereich 6 infolge der abgeblasenen Luftmenge im Anschluss an den Hustenreiz den Drucksollwert unterschreitet.

In Verbindung mit dem Absperrelement 46 ist es gemäß einer besonders bevorzugten Ausführungsform der Erfindung von Vorteil, wenn der Überdruckbereich 6 mit einem Expansionsbehältnis 48 in bevorzugt unmittelbarer Strömungsverbindung steht, welches bei einem Überschreiten eines Druck-Schwellenwertes, der oberhalb des gewünschten Drucksollwertes oder Öffnungsdrucks des Überdruckventils 30 liegt, unter Erweiterung seines Volumens expandiert wird. So kann der Druck-Schwellenwert beispielsweise 30% oder mehr über dem Drucksollwert liegen, bei welchem das Ventil 30 öffnet.

Durch den Einsatz eines zuvor beschriebenen Expansionsbehältnisses, welches bevorzugt als ein Ballon 48 aus gummielastischem Werkstoff ausgestaltet ist, ergibt sich der Vorteil, dass sich der Cuff aufgrund des über dem Drucksollwert liegenden Druck-Schwellenwerts des Expansionsverhältnisses 48 bequem mit einer handelsüblichen Spritze 14 befüllen lässt, ohne dass sich das Volumen des Expansionsbehältnises 48 hierbei nennenswert vergrößert. Gleichzeitig wird jedoch im Falle eines Hustenreizes sichergestellt, dass der hierbei kurzzeitig durch ein Zusammenziehen der Trachea entstehende Überdruckstoß im Überdruckbereich 6 vom Expansionsbehältnis 48 aufgenommen wird, das sich nach Beendigung des Hustenreizes aufgrund seiner Eigenelastizität wieder auf seine ursprüngliche Größe zusammenzieht. Durch das selbständige Zusammenziehen gelangt die aus dem Expansionsbehältnis entweichende Luftmenge über den Zuleitungsschlauch 2 wieder zurück in den Cuff und stellt dort den gewünschten Drucksollwert, mit welchem der Cuff an der Innenwand der Trachea anliegt wieder her. Das Absperrelement 46 ist hierbei
- wie in Fig. 6 gezeigt - in vorteilhafter Weise geschlossen und die Spritze 14 von den einlassseitigen Anschlussmitteln 12a abgezogen.

### Liste der Bezugszeichen

- 1: erfindungsgemäßer Befülladapter
- 2: Zuleitungsschlauch
- 4: Gehäuse
- 6: Überdruckbereich
- 8a: schlauchseitiger weiblicher Anschlusskonnektor
- 8b: konischer männlicher Anschlusskonnektor
- 10: erstes Ventil
- 12a: einlassseitige zweite Anschlussmittel/konischer weiblicher Anschlusskonnektor
- 12b: konischer männlicher Anschlusskonnektor einer Spritze
- 14: mechanisch betätigte Luftfördereinrichtung/Einwegspritze
- 14a: Kolben der Spritze
- 16: Bohrung im Gehäuse
- 18: zylindrischer Hohlkörper
- 20: Innenraum des zylindrischen Hohlkörpers
- 22: Platte/Flansch
- 24: radiale Durchgangsbohrung
- 26: innere Wandfläche der Bohrung im Gehäuse
- 28: federelastische Mittel/Spiraldruckfeder des ersten Ventils
- 30: Überdruckventil
- 32: Öffnung
- 34: Plättchen/Membran
- 36: federelastische Mittel/Spiraldruckfeder des Überdruckventils
- 38: Wandung
- 40: Gehäuse des Überdruckventils
- 42: Überwurfmutter
- 44: Warneinrichtung
- 44a: Plättchen/Membranzungen

- 46: Absperrelement/Auf-Zu-Ventil
- 46a: Hebel
- 48: Expansionsbehältnis/Ballon

## Patentansprüche

1. Befülladapter (1) zum Befüllen eines Cuffs, der insbesondere an einem in die menschliche Trachea einsetzbaren Tubus zur Abdichtung desselben angeordnet und über einen Zuleitungsschlauch (2) mit Luft beaufschlagbar ist,
**gekennzeichnet durch**
ein nach außen hin geschlossenes Gehäuse (4) mit einem darin definierten Überdruckbereich (6), erste mit dem Überdruckbereich (6) in Strömungsverbindung stehende und mit dem Zuleitungsschlauch (2) druckdicht verbindbare auslassseitige Anschlussmittel (8a, 8b), zweite einlassseitige Anschlussmittel (12a, 12b), die über eine manuell betätigte Lunfordereinrichtung, insbesondere eine Spritze (14), mit Überdruck beaufschlagbar sind und die mit dem Überdruckbereich (6) über ein erstes Ventil (10) strömungmäßig verbindbar sind, sowie ein mit dem Überdruckbereich (6) strömungsmäßig verbundenes Überdruckventil (30), welches den Überdruckbereich (6) beim Überschreiten eines vorgegebenen Drucksollwerts entlüftet.

2. Adapter nach Anspruch 1,
**dadurch gekennzeichnet, dass**
das Überdruckventil (30) ein federbelastetes Rückschlagventil ist.

3. Adapter nach Anspruch 2,
**dadurch gekennzeichnet, dass**
Druck-Verstellmittel (36, 42) vorgesehen sind, über die die Größe des Drucksollwerts, bei welchem das Überdruckventil (30) öffnet und den Überdruckbereich (6) entlüftet, veränderbar ist.

4. Adapter nach einem der vorhergehenden Ansprüche,
**dadurch gekennzeichnet, dass**
das Überdruckventil (30) mit einer Warneinrichtung (44) in Verbindung steht, welche beim Öffnen des Überdruckventils (30) betätigt wird.

5. Adapter nach Anspruch 4,
**dadurch gekennzeichnet, dass**
die Warneinrichtung (44) eine mit dem Überdruckventil (30) strömungsmäßig verbundene akustische Warneinrichtung (44a) umfasst, welche beim Öffnen des Überdruckventils von der aus dem Überdruckbereich (6) entweichenden Luft unter Erzeugung eines Pfeiftons durchströmt wird.

6. Adapter nach einem der vorhergehenden Ansprüche,
**dadurch gekennzeichnet, dass**
dem Überdruckventil (30) ein Absperrelement (46) zugeordnet ist, mittels welchem eine Entlüftung des Überdruckbereichs (6) auch bei einem Überschreiten des vorgegebenen Drucks verhindert wird.

7. Adapter nach Anspruch 6,
**dadurch gekennzeichnet, dass**
das Absperrelement (46) ein dem Überdruckventil strömungsmäßig vor- oder nachgeordnetes Auf-Zu-Ventil (46) ist.

8. Adapter nach einem der vorhergehenden Ansprüche,
**dadurch gekennzeichnet, dass**
der Überdruckbereich (6) mit einem Expansionsbehältnis (48) in Strömungsverbindung steht, welches bei einem Überschreiten eines oberhalb des Öffnungsdrucks des Überdruckventils (30) liegenden bruck-Schwellenwerts unter Erweiterung seines Volumens expandierbar ist.

9. Adapter nach Anspruch 8,
**dadurch gekennzeichnet, dass**
das Expansionsbehältnis einen Ballon (48) aus einem gummielastischen Werkstoff umfasst.

10. Adapter nach einem der vorhergehenden Ansprüche,
**dadurch gekennzeichnet, dass**
das erste Ventil (10) einen in einer Bohrung (16) des Gehäuses (4) verschiebbar aufgenommenen zylindrischen Hohlkörper (18) umfasst, dessen Innenraum (20) an seinem einen Ende im Bereich des Überdruckbereichs (6) stirnseitig verschlossen ist, und das an seinem anderen Ende mit den zweiten einlassseitigen Anschlussmitteln (12a) in Strömungsverbindung steht, wobei der zylindrische Hohlkörper (18) eine im Abstand vom ersten Ende angeordnete radiale Durchgangsbohrung (24) aufweist und durch sich am Gehäuse (4) abstützende federelastische Mittel (28) in eine Schließstellung gedrängt wird, in der die radiale Durchgangsbohrung (24) durch eine innere Wandfläche (26) der Bohrung (16) verschlossen wird.
